# EUROPEAN PATENT APPLICATION

(11) **EP 2 659 970 A1**
(43) Date of publication of application: **06.11.2013**
(21) Application number: 11852815.7
(22) Date of filing: 28.12.2011
(51) Int. Cl.: B01J 29/40, C07C 4/06, C07C 15/04, C07C 15/06, C07C 15/067, C10G 11/05, C07B 61/00

(54) **CATALYST FOR PRODUCING MONOCYLIC AROMATIC HYDROCARBON AND METHOD FOR PRODUCING MONOCYCLIC AROMATIC HYDROCARBON**

(30) Priority: 28.12.2010 JP 2010294184
(71) Applicant: JX Nippon Oil & Energy Corporation, Chiyoda-ku Tokyo 100-8162 (JP)
(72) Inventor: YANAGAWA, Shinichiro, Tokyo 100-8162 (JP); KOBAYASHI, Masahide, Tokyo 100-8162 (JP); IWASA, Yasuyuki, Tokyo 100-8162 (JP); IDA, Ryoji, Tokyo 100-8162 (JP)
(74) Representative: Elend, Almut Susanne
(86) International application number: PCT/JP2011/080468
(87) International publication number: WO 2012/091119

(57) **Abstract**

The catalyst for producing monocyclic aromatic hydrocarbons is for producing monocyclic aromatic hydrocarbons having 6 to 8 carbon number from oil feedstock having a 10 volume% distillation temperature of 140°C or higher and a 90 volume% distillation temperature of 380°C or lower. The catalyst contains crystalline aluminosilicate and a rare earth element, in which the amount of the rare earth element expressed in terms of the element is 0.1 to 10 mass% based on the crystalline aluminosilicate. In the production method of monocyclic aromatic hydrocarbons, oil feed stock having a 10 volume% distillation temperature of 140°C or higher and a 90 volume% distillation temperature of 380°C or lower is brought into contact with the catalyst for producing monocyclic aromatic hydrocarbons.

## Description

### TECHNICAL FIELD

The present invention relates to a catalyst for producing monocyclic aromatic hydrocarbons and a production method of monocyclic aromatic hydrocarbons.
Priority is claimed on Japanese Patent Application No. 2010-294184, filed December 28, 2010, the content of which is incorporated herein by reference.

### BACKGROUND ART

Light Cycle Oil (hereinafter, called "LCO") as cracked light oil that is generated by a fluidized catalytic cracking contains a large amount of polycyclic aromatic hydrocarbon and is used as light oil or heavy oil. However, in recent years, investigations have been conducted to obtain, from LCO, monocyclic aromatic hydrocarbons having 6 to 8 carbon numbers (for example, benzene, toluene, xylene, ethylbenzene and the like), which can be utilized as high octane value gasoline base materials or petrochemical feedstocks and have a high added value.
For example, Patent Documents 1 to 3 suggest methods for producing monocyclic aromatic hydrocarbons from polycyclic aromatic hydrocarbons that are contained in LCO and the like in a large amount, by using a zeolite catalyst.
However, Patent Documents 1 to 3 do not disclose that when monocyclic aromatic hydrocarbons having 6 to 8 carbon number are produced using the catalyst disclosed in Patent Documents 1 to 3, the yield of monocyclic aromatic hydrocarbons having 6 to 8 carbon number is sufficiently high at the initial stage of reaction. Moreover, the yield of monocyclic aromatic hydrocarbons having 6 to 8 carbon number was low in a normal state.

When monocyclic aromatic hydrocarbons are produced from heavy oil feedstock containing polycyclic aromatic hydrocarbons, catalyst regeneration for removing a carbonaceous substance needs to be performed with a high frequency since a large amount of carbonaceous substance is precipitated on the catalyst and rapidly decreases the activity. Moreover, when a circulating fluidized bed for performing a process of efficiently repeating reaction-catalyst regeneration is employed, the temperature for catalyst regeneration needs to be higher than the reaction temperature, so the temperature environment of the catalyst becomes more severe.
When a zeolite catalyst is used as a catalyst under such a severe condition, hydrothermal deterioration of the catalyst progresses, and the yield of monocyclic aromatic hydrocarbons in a normal state decreases. Accordingly, the improvement of hydrothermal stability is required for the catalyst. However, for the zeolite catalyst disclosed in Patent Documents 1 to 3, a measure for improving hydrothermal stability was not taken, and the practical usefulness thereof was extremely low.

As the method for improving hydrothermal stability, a method using zeolite having a high Si/Al ratio, a method of stabilizing a catalyst by performing hydrothermal treatment in advance, such as in USY-type zeolite, a method of adding phosphorus to zeolite, a method of adding a rare earth metal to zeolite, a method of improving a structure directing agent at the time of zeolite synthesis, and the like are known.

### Prior art documents

### Patent documents

[Patent Document 1] Japanese Unexamined Patent Application, First Publication No. H3-2128
[Patent Document 2] Japanese Unexamined Patent Application, First Publication No. H3-52993
[Patent Document 3] Japanese Unexamined Patent Application, First Publication No. H3-26791

### DISCLOSURE OF INVENTION

### Problems to be solved by the invention

An object of the present invention is to provide a catalyst for producing monocyclic aromatic hydrocarbons that can produce monocyclic aromatic hydrocarbons having 6 to 8 carbon number with a high yield from oil feedstock containing polycyclic aromatic hydrocarbons not only at the initial stage of reaction but also in a normal state, and a production method of monocyclic aromatic hydrocarbons.

### Means to solve the problems

[1] A catalyst for producing monocyclic aromatic hydrocarbons that is for producing monocyclic aromatic hydrocarbons having 6 to 8 carbon number from oil feedstock having a 10 volume% distillation temperature of 140°C or higher and a 90 volume% distillation temperature of 380°C or lower, the catalyst including crystalline aluminosilicate and a rare earth element, in which the amount of the rare earth element that is expressed in terms of the element is 0.1 to 10 mass% based on the crystalline aluminosilicate.
[2] A catalyst for producing monocyclic aromatic hydrocarbons that is for producing monocyclic aromatic hydrocarbons having 6 to 8 carbon number from oil feedstock having a 10 volume% distillation temperature of 140°C or higher and a 90 volume% distillation temperature of 380°C or lower, the catalyst including crystalline aluminosilicate, a binder, and a rare earth element, in which the amount of the rare earth element that is expressed in terms of the element is 0.1 to 30 mass% based on the weight of the catalyst.
[3] The catalyst for producing monocyclic aromatic hydrocarbons according to [1] or [2], further including gallium and/or zinc.
[4] The catalyst for producing monocyclic aromatic hydrocarbons according to [3], in which the amount of gallium and/or zinc is 0.05 to 2 mass% based on the crystalline aluminosilicate.
[5] The catalyst for producing monocyclic aromatic hydrocarbons according to [3] or [4], in which the amount of gallium and/or zinc is 0.02 to 2 mass% based on the weight of the catalyst.
[6] The catalyst for producing monocyclic aromatic hydrocarbons according to any one of [1] to [5], in which the crystalline aluminosilicate contains phosphorus, and the amount of phosphorus contained in the crystalline aluminosilicate is 0.1 to 3.5 mass% based on the crystalline aluminosilicate.
[7] The catalyst for producing monocyclic aromatic hydrocarbons according to [1] to [6], further including phosphorus, in which the amount of phosphorus is 0.1 to 10 mass% based on the weight of the catalyst.
[8] The catalyst for producing monocyclic aromatic hydrocarbons according to any one of [1] to [7], in which the rare earth element is at least one kind selected from the group consisting of lanthanum, cerium, praseodymium, neodymium, samarium, gadolinium, and dysprosium.
[9] The catalyst for producing monocyclic aromatic hydrocarbons according to any one of [1] to [8], in which the crystalline aluminosilicate is medium pore size zeolite.
[10] The catalyst for producing monocyclic aromatic hydrocarbons according to any one of [1] to [9], in which the crystalline aluminosilicate is MFI type zeolite.
[11] A production method of monocyclic aromatic hydrocarbons having 6 to 8 carbon number, including a step of bringing oil feedstock having a 10 volume% distillation temperature of 140°C or higher and a 90 volume% distillation temperature of 380°C or lower into contact with the catalyst for producing monocyclic aromatic hydrocarbons according to any one of [1] to [10].
[12] The production method of monocyclic aromatic hydrocarbons having 6 to 8 carbon number according to [11], in which the oil feedstock contains light cycle oil generated by a fluidized catalytic cracking.
[13] The production method of monocyclic aromatic hydrocarbons having 6 to 8 carbon number according to [11] or [12], in which the oil feedstock is brought into contact with the catalyst for producing monocyclic aromatic hydrocarbons by using a fluidized-bed reaction equipment.

### Effect of the invention

According to the catalyst for producing monocyclic aromatic hydrocarbons and the production method of monocyclic aromatic hydrocarbons having 6 to 8 carbon number of the present invention, it is possible to produce monocyclic aromatic hydrocarbons having 6 to 8 carbon number with a high yield from oil feedstock containing polycyclic aromatic hydrocarbons. Moreover, even in a normal state, the yield of monocyclic aromatic hydrocarbons having 6 to 8 carbon number becomes high.

### BEST MODE FOR CARRYING OUT THE INVENTION

Hereinafter, an embodiment of the catalyst for producing monocyclic aromatic hydrocarbons and the production method of monocyclic aromatic hydrocarbons having 6 to 8 carbon number of the present invention will be described.

### First embodiment:

### (Catalyst for producing monocyclic aromatic hydrocarbons)

A catalyst for producing monocyclic aromatic hydrocarbons (hereinafter, abbreviated to a "catalyst") of a first embodiment is for producing monocyclic aromatic hydrocarbons having 6 to 8 carbon number (hereinafter, abbreviated to "monocyclic aromatic hydrocarbons") from oil feedstock containing polycyclic aromatic hydrocarbons and saturated hydrocarbons and contains crystalline aluminosilicate and a rare earth element.

### [Crystalline aluminosilicate]

The crystalline aluminosilicate is not particularly limited, but is preferably, for example, pentasil type zeolite or medium pore size zeolite. As the medium pore size zeolite, zeolites having an MFI, MEL, TON, MTT, MRE, FER, AEL, or EUO type crystal structure are more preferable. Particularly, zeolites having an MFI and/or MEL type crystal structure are preferable since they further increase the yield of monocyclic aromatic hydrocarbons.
The zeolites of MFI type, MEL type, and the like belong to zeolites having known types of structures that are publicly introduced by The Structure Commission of the International Zeolite Association (Atlas of Zeolite Structure Types, W. M. Meiyer and D. H. Olson (1978), Distributed by Polycrystal Book Service, Pittsburgh, PA, USA).
Provided that the total amount of the catalyst is 100 mass%, the amount of the crystalline aluminosilicate in the catalyst is preferably 10 to 100 mass%, more preferably 60 to 100 mass%, even more preferably 70 to 100 mass%, and particularly preferably 90 to 100 mass%. If the amount of the crystalline aluminosilicate is from 10 mass% or more, a sufficiently high degree of catalytic activity is obtained.
In view of increasing the yield of monocyclic aromatic hydrocarbons, a molar ratio between silicon and aluminum (Si/Al ratio) in the crystalline aluminosilicate is preferably from 10 to 500. The upper limit of the ratio is more preferably 300 or less, and more preferably 250 or less. If the Si/Al ratio of the crystalline aluminosilicate exceeds 500, this is not preferable since the yield of monocyclic aromatic hydrocarbons decreases.

### [Rare earth element]

As forms of the rare earth element in the catalyst of the present embodiment, there are a form in which the rare earth element is incorporated into the lattice skeleton of crystalline aluminosilicate, a form in which the rare earth element is contained in crystalline aluminosilicate, and a form as a combination of both of them, for example.
The catalyst in which the rare earth element is incorporated into the lattice skeleton of crystalline aluminosilicate has a structure in which SiO₄, AlO₄, and the rare earth element structure form tetrahedral coordination in the skeleton. Moreover, the catalyst in which the rare earth element is incorporated into the lattice skeleton of crystalline aluminosilicate is formed by, for example, ion exchange in which the crystalline aluminosilicate is mixed with an aqueous solution containing the rare earth element so as to substitute a portion inside the skeleton of crystalline aluminosilicate with the rare earth element, a method of using a crystallization accelerator containing the rare earth element during zeolite synthesis, and the like.
The catalyst in which the rare earth element is contained in crystalline aluminosilicate is obtained by adding the rare earth element to crystalline aluminosilicate by known methods such as ion exchange and impregnation. The source of the rare earth element used at this time is not particularly limited, and examples thereof include those prepared by dissolving an aqueous nitric acid solution containing the rare earth element, an aqueous solution containing a metal salt of the rare earth element, or the like in water at any concentration.

In the catalyst of the present embodiment, provided that the total mass of the crystalline aluminosilicate is 100 mass%, the amount of the rare earth element contained in the crystalline aluminosilicate is 0.1 to 10 mass% that is expressed in terms of the element. Moreover, the upper limit of the content is more preferably 9 mass% or less, and more preferably 8 mass% or less. If the amount of the rare earth element contained in the crystalline aluminosilicate is 0.1 mass% or more, the reduction in the yield of monocyclic aromatic hydrocarbons caused over time can be prevented, and if it is 10 mass% or less, the yield of monocyclic aromatic hydrocarbons can be increased.

The rare earth element in the catalyst of the present embodiment is at least one kind selected from the group consisting of lanthanum (La), cerium (Ce), praseodymium (Pr), neodymium (Nd), samarium (Sm), gadolinium (Gd), and dysprosium (Dy). One kind of these can be used alone, or plural kinds thereof can be used in combination. Among these rare earth elements, cerium (Ce) and Lanthanum (La) are more preferable.

### [Phosphorus]

In the catalyst of the present embodiment, the crystalline aluminosilicate may contain phosphorus. Provided that the total mass of the crystalline aluminosilicate is 100 mass%, the amount of phosphorus contained in the crystalline aluminosilicate in the catalyst of the present embodiment is preferably 0.1 to 3.5 mass%. Moreover, the lower limit of the content is more preferably 0.2 mass% or more, and the upper limit thereof is more preferably 3.0 mass% or less and particularly preferably 2.8 mass% or less. If the amount of phosphorus contained in the crystalline aluminosilicate is 0.1 mass% or more, the reduction in the yield of monocyclic aromatic hydrocarbons caused over time can be prevented, and if it is 3.5 mass% or less, the yield of monocyclic aromatic hydrocarbons can be increased.

The method of adding phosphorus to the catalyst of the present embodiment is not particularly limited, and examples thereof include a method of adding a phosphorus compound to crystalline aluminosilicate, crystalline aluminogallosilicate, or crystalline aluminozincosilicate by ion exchange, impregnation, or the like so as to cause phosphorus to be supported on crystalline aluminosilicate, a method of replacing a portion of the inside of the crystalline aluminosilicate skeleton with phosphorus by adding a phosphorus compound during zeolite synthesis, a method of using a phosphorus-containing crystallization accelerator during zeolite synthesis, and the like. An aqueous phosphate ion-containing solution used at this time is not particularly limited, and it is possible to preferably use solutions that are prepared by dissolving phosphoric acid, diammonium hydrogen phosphate, ammonium dihydrogen phosphate, other water-soluble phosphate, and the like in water at any concentration.

### [Gallium and zinc]

The catalyst according to the present embodiment may contain gallium and/or zinc. As forms of the catalyst according to the present embodiment that contain gallium and/or zinc, there are the form in which gallium and/or zinc are (is) incorporated into the lattice skeleton of crystalline aluminosilicate (crystalline aluminogallosilicate and/or crystalline aluminozincosilicate), the form in which gallium is supported on crystalline aluminosilicate (gallium-supported crystalline aluminosilicate) and/or the form in which zinc is supported on crystalline aluminosilicate (zinc-supported crystalline aluminosilicate), and the form as a combination of both of them, for example.
The crystalline aluminogallosilicate has a structure in which SiO₄, AlO₄, and GaO₄ structures form tetrahedral coordination in the skeleton, and the crystalline aluminozincosilicate has a structure in which SiO₄, AlO₄, and ZnO₄ structures form tetrahedral coordination in the skeleton. Moreover, the crystalline aluminogallosilicate and/or crystalline aluminozincosilicate are (is) obtained by, for example, crystallization of gel by hydrothermal synthesis, a method of inserting gallium and/or zinc into the lattice skeleton of crystalline aluminosilicate, and a method of inserting aluminum into the lattice skeleton of crystalline gallosilicate and/or crystalline aluminozincosilicate.
The gallium-supported crystalline aluminosilicate and/or zinc-supported crystalline aluminosilicate are (is) a substance in which gallium and/or zinc are (is) supported on crystalline aluminosilicate by a known method such as ion exchange, impregnation, or the like. Examples of the source of gallium or zinc used at this time are not particularly limited and include gallium salts such as gallium nitrate and gallium chloride, gallium oxide, zinc salts such as zinc nitrate and zinc chloride, zinc oxide, and the like.

Provided that the total mass of the crystalline aluminosilicate according to the present embodiment is 100 mass%, the amount of gallium and/or zinc in the catalyst is preferably 0.05 to 2.0 mass%, and the lower limit of the content is more preferably 0.1 mass% or more. The upper limit thereof is more preferably 1.6 mass% or less, and particularly preferably 1.0 mass% or less. If the amount of gallium and/or zinc supported on crystalline aluminosilicate in the catalyst is 0.05 mass% or more, the reduction in the yield of monocyclic aromatic hydrocarbons caused over time can be prevented, and if the content is 2.0 mass% or less, the yield of monocyclic aromatic hydrocarbons can be increased.
Provided that the total mass of the catalyst is 100 mass%, the amount of gallium and/or zinc in the catalyst of the present embodiment is preferably 0.02 to 2 mass%, and the lower limit of the content is more preferably 0.05 mass% or more. The upper limit thereof is more preferably 1.6 mass% or less, and particularly preferably 1.2 mass% or less. Provided that the total mass of the catalyst is 100 mass%, if the amount of gallium and/or zinc contained in the catalyst is 0.02 mass% or more, the reduction in the yield of monocyclic aromatic hydrocarbons caused over time can be prevented, and if the content is 2 mass% or less, the yield of monocyclic aromatic hydrocarbons can be increased.

The catalyst according to the present embodiment may be either a catalyst containing gallium or zinc individually or a catalyst containing both of them. Moreover, the catalyst may further contain other metals in addition to gallium and/or zinc.

The catalyst of the present embodiment is obtained by calcining (calcining temperature of 300 to 900°C) crystalline aluminosilicate, crystalline aluminogallosilicate, or crystalline aluminozincosilicate that contains a rare earth element or phosphorus, or crystalline aluminosilicate containing a rare earth element, gallium and/or zinc, and phosphorus as described above.

### Second embodiment:

### (Catalyst for producing monocyclic aromatic hydrocarbons)

A catalyst for producing monocyclic aromatic hydrocarbons (hereinafter, abbreviated to a "catalyst") of a second embodiment is for producing monocyclic aromatic hydrocarbons having 6 to 8 carbon number (hereinafter, abbreviated to "monocyclic aromatic hydrocarbons") from oil feedstock containing polycyclic aromatic hydrocarbons and saturated hydrocarbons, and contains crystalline aluminosilicate, a binder, and a rare earth element.

### [Crystalline aluminosilicate]

As the crystalline aluminosilicate of the present embodiment, the same crystalline aluminosilicate as that of the first embodiment can be used.
Provided that the total amount of the catalyst is 100 mass%, the amount of the crystalline aluminosilicate in the catalyst is preferably 10 to 95 mass%, more preferably 20 to 80 mass%, even more preferably 25 to 70 mass%, and still more preferably 35 to 60 mass%. If the amount of the crystalline aluminosilicate is from 10 mass% to 95 mass%, a sufficiently high degree of catalytic activity is obtained.
A molar ratio between silicon and aluminum (Si/Al ratio) in the crystalline aluminosilicate may be the same as that of the first embodiment.

### [Rare earth element]

As a rare earth element of the present embodiment, the same rare earth element as that of the first embodiment can be used.
As forms of the rare earth metal, for example, there are the form in which a rare earth element is incorporated into the lattice skeleton of crystalline aluminosilicate, the form in which a rare earth element is contained in crystalline aluminosilicate, and the form as a combination of both of them, similarly to the first embodiment.

The amount of the rare earth element in the catalyst of the present embodiment that contains a binder (such as an inorganic oxide) is 0.1 to 30 mass% based on the weight of the catalyst, in terms of the element. Moreover, the upper limit of the content is more preferably 25 mass% or less, and particularly preferably 20 mass% or less. If the amount of the rare earth element based on the total weight of the catalyst is 0.1 mass% or more, the reduction in the yield of monocyclic aromatic hydrocarbons caused over time can be prevented, and if it is 30 mass% or less, the yield of monocyclic aromatic hydrocarbons can be increased.

### [Binder]

Examples of the binder of the present embodiment include inorganic oxides. Specific examples thereof include silica, alumina, zirconia, titania, a mixture of these, and the like. Among these, silica and alumina are particularly preferable. If the catalyst contains a binder, moldability or strength is improved, and the catalyst becomes durable regarding long-term use. Moreover, when the catalyst is used in a fluidized bed, the binder is indispensable for improving fluidity or the like.
Provided that the total mass of the catalyst is 100 mass%, the amount of the binder contained in the catalyst is preferably 5 to 90 mass%, more preferably 10 to 80 mass%, and even more preferably 25 to 75 mass%.
Optionally, a binder containing a rare earth element or phosphorus can be used.

### [Phosphorus]

In the catalyst of the present embodiment, crystalline aluminosilicate may contain phosphorus similarly to the first embodiment.

In the present embodiment in which the catalyst contains a binder, the amount of phosphorus is preferably 0.1 to 10 mass% based on the total weight of the catalyst. Moreover, the lower limit of the content is more preferably 0.5 mass% or more, and the upper limit thereof is more preferably 9 mass% or less, and particularly preferably 8 mass% or less. If the amount of phosphorus based on the total weight of the catalyst is 0.1 mass% or more, the reduction in the yield of monocyclic aromatic hydrocarbons caused over time can be prevented, and if it is 10 mass% or less, the yield of monocyclic aromatic hydrocarbons can be increased.

### [Gallium and zinc]

The catalyst of the present embodiment may contain gallium and/zinc that are (is) in the same form as in the first embodiment.
The catalyst of the present embodiment may be either a catalyst containing gallium or zinc individually or a catalyst containing both of them. Moreover, the catalyst may further contain other metals in addition to gallium and/or zinc.
The amount of gallium and/or zinc in the catalyst of the present embodiment may be the same as in the first embodiment.

In the present embodiment in which the catalyst contains a binder, a binder may be mixed with crystalline aluminosilicate, and then gallium and/or zinc, a rare earth element, and phosphorus may optionally be added to the mixture to produce the catalyst. Moreover, in the present embodiment in which the catalyst contains a binder, a binder may be mixed with crystalline aluminosilicate supporting gallium and/or zinc, or a binder may be mixed with crystalline aluminogallosilicate and/or crystalline aluminozincosilacate, and then a rare earth element and phosphorus may be added thereto to produce the catalyst.

The catalyst of the present embodiment is obtained by calcining (calcining temperature of 300 to 900°C) a mixture including the binder and a zeolite slurry which contains crystalline aluminosilicate, crystalline aluminogallosilicate, or crystalline aluminozincosilicate that contains a rare earth element or phosphorus, or crystalline aluminosilicate containing a rare earth element, gallium and/or zinc, and phosphorus as described above.

### [Shape]

The catalyst according to the first and second embodiments is shaped into, for example, powder, granules, pellets, and the like, according to the reaction mode.
For example, the catalyst is shaped into powder in the case of a fluidized bed and shaped into granules or pellets in the case of a fixed bed. An average particle size of the catalyst used in a fluidized bed is preferably 30 to 180 µm, and more preferably 50 to 100 µm. Moreover, a bulk density of the catalyst used in a fluidized bed is preferably 0.4 to 1.8 g/cc, and more preferably 0.5 to 1.0 g/cc.
The average particle size indicates a size of particles accounting for 50 mass% in a particle size distribution obtained by classification performed by sieving, and the density is a value measured by the method of JIS standard R9301-2-3.
In order to obtain the catalyst having a granule or pellet form, an inactive oxide as a binder may be optionally mixed into crystalline aluminosilicate or a catalyst, and then the resultant may be molded using various molding machines.
In addition, the catalyst according to the second embodiment is not restricted in terms of the form of reaction, but is preferably used in a fluidized bed.

### (Production method of monocyclic aromatic hydrocarbons)

The production method of monocyclic aromatic hydrocarbons according to the first and second embodiments is a method of bringing oil feedstock into contact with each of the catalysts of the above respective embodiments to undergo a reaction.
The present reaction is a method in which the oil feedstock is allowed to come into contact with an acid point of the catalyst to undergo various reactions such as cracking, dehydrogenation, cyclization, and hydrogen transfer, whereby polycyclic aromatic hydrocarbons undergo ring opening and are converted into monocyclic aromatic hydrocarbons.

### [Oil feedstock]

The oil feedstock used in the present embodiment is oil having a 10 volume% distillation temperature of 140°C or higher and a 90 volume% distillation temperature of 380°C or lower. If oil having a 10 volume% distillation temperature of lower than 140°C is used, BTX (benzene, toluene, and xylene) is produced from light oil, and this does not fit into the main object of the present embodiment. Accordingly, the 10 volume% distillation temperature of the oil is preferably 140°C or higher, and more preferably 150°C or higher. Moreover, when oil feedstock having a 90 volume% distillation temperature of higher than 380°C is used, the amount of coke deposited onto the catalyst increases, whereby the catalytic activity tends to be rapidly reduced. Accordingly, the 90 volume% distillation temperature of the oil feedstock is preferably 380°C or lower, and more preferably 360°C or lower.
In addition, the 10 volume% distillation temperature, 90 volume% distillation temperature, and endpoint described herein are values measured based on JIS K2254 "Petroleum products-Determination of distillation characteristics".
Examples of the oil feedstock having a 10 volume% distillation temperature of 140°C or higher and a 90 volume% distillation temperature of 380°C or lower include Light Cycle Oil (LCO) generated by a fluidized catalytic cracking, coal-liquefied oil, hydrocracked and refined heavy oil, straight-run kerosene, straight-run light oil, coker kerosene, coker light oil, hydrocracked and refined oil sand, and the like. Among these, Light Cycle Oil (LCO) generated by a fluidized catalytic cracking is more preferable.
If the oil feedstock contains a large amount of polycyclic aromatic hydrocarbons, the yield of monocyclic aromatic hydrocarbons having 6 to 8 carbon number decreases. Accordingly, the amount of polycyclic aromatic hydrocarbons (polycyclic aromatic fraction) in the oil feedstock is preferably 50 volume% or less, and more preferably 30 volume% or less.
In addition, the polycyclic aromatic fraction described herein refers to the sum of the amount of bicyclic aromatic hydrocarbons (bicyclic aromatic fraction) and the amount of aromatic hydrocarbons having three or more rings (aromatic fraction having three or more rings) that are measured based on JPI-5S-49 "Petroleum products-Determination of hydrocarbon types-High performance liquid chromatography".

### [Form of reaction]

As the form of reaction at the time when the oil feedstock is brought into contact with the catalyst and reacted, a fixed bed, a moving bed, a fluidized bed, and the like are exemplified. In the present embodiment, a heavy fraction is used as oil feedstock. Accordingly, a fluidized bed that makes it possible to continuously remove the fraction of coke attached to the catalyst and to stably carry out the reaction is preferable. Particularly, a continuous regeneration-type fluidized bed that can cause the catalyst to circulate between a reactor and regenerator and can continuously repeat reaction-regeneration is preferable. It is preferable that the oil feedstock be brought into contact with the catalyst be in a gaseous state. Moreover, the oil feedstock may be optionally diluted with gas, and when unreacted oil is generated, this may be optionally recycled.

### [Reaction temperature]

The reaction temperature at the time when the oil feedstock is brought into contact with the catalyst and reacted is not particularly limited, and is preferably 350°C to 700°C. The lower limit of the temperature is more preferably 450°C or lower since sufficient reaction activity is obtained. On the other hand, the upper limit thereof is more preferably 650°C or lower since this temperature is advantageous in view of energy and makes it possible to easily regenerate the catalyst.

### [Reaction pressure]

The reaction pressure at the time when the oil feedstock is brought into contact with the catalyst and reacted is preferably 1.5 MPaG or lower, and more preferably 1.0 MPaG or lower. If the reaction pressure is 1.5 MPaG or lower, it is possible to prevent light gas from being additionally generated and to diminish pressure resistance of the reaction apparatus. Though not particularly limited, the lower limit of the reaction pressure is preferably equal to or higher than normal pressure in view of cost and the like.

### [Contact time]

The time for which the oil feedstock comes into contact with the catalyst is not particularly limited as long as a substantially desired reaction is caused. For example, the contact time is preferably 1 second to 300 seconds in terms of the time required for gas on the catalyst to pass. The lower limit of the contact time is more preferably 5 seconds or longer, and the upper limit thereof is more preferably 150 seconds or shorter. If the contact time is 1 second or longer, the reaction can proceed reliably, and if it is 300 seconds or shorter, it is possible to inhibit a carbonaceous substance from being accumulated onto the catalyst by coking or the like, and to suppress the amount of light gas generated by cracking.

In the production method of monocyclic aromatic hydrocarbons of the present embodiment, the oil feedstock is brought into contact with an acid point of the catalyst to undergo various reactions such as cracking, dehydrogenation, cyclization, and hydrogen transfer and cause ring opening of polycyclic aromatic hydrocarbons, thereby obtaining monocyclic aromatic hydrocarbons.
In the present embodiment, the yield of monocyclic aromatic hydrocarbons at the initial state of reaction is preferably 25 mass% or more, more preferably 30 mass% or more, and even more preferably 35 mass% or more.
Moreover, the yield of monocyclic aromatic hydrocarbons in a normal state is preferably 20 mass% or more, more preferably 25 mass% or more, and even more preferably 30 mass% or more.
If the yield of monocyclic aromatic hydrocarbons at the initial stage of reaction is less than 25 mass%, and the yield of monocyclic aromatic hydrocarbons in a normal state is less than 20 mass%, this is not preferable since the concentration of monocyclic aromatic hydrocarbons in a product is low, and the collecting efficiency is lowered.

The production method of the present embodiment described above uses the catalyst described above. Accordingly, at the initial stage of reaction and in a normal state, monocyclic aromatic hydrocarbons can be produced with a high yield.

### EXAMPLES

Hereinafter, the present invention will be described in more detail based on examples and comparative examples, but the present invention is not limited to these examples.

### <Preparation of catalyst>

### (Example 1)

30 g of proton-type crystalline aluminosilicate that had an MFI structure and a molar ratio of silicon/aluminum (Si/Al ratio) of 35 was impregnated with an aqueous cerium (III) nitrate solution such that 2.5 mass% (value calculated when the total mass of the crystalline aluminosilicate is regarded as being 100 mass%) of cerium was contained in the proton-type crystalline aluminosilicate, and the resultant was heated at 80°C under stirring to cause ion exchange between cerium ions and protons of aluminum, followed by drying at 120°C. Thereafter, the resultant was calcined for 3 hours at 780°C under an air flow, thereby obtaining a catalyst including cerium-containing crystalline aluminosilicate.
A pressure of 39.2 MPa (400 kgf) was applied to the obtained catalyst to form tablets, and the resultant was coarsely pulverized to have a size of 20 to 28 mesh (average particle size of 0.65 to 0.85 mm), thereby obtaining a granular catalyst 1 (hereinafter, called a "granulated catalyst 1"). In the granulated catalyst 1, the Si/Al ratio was 35, and the amount of cerium (based on the total amount of the catalyst) was 2.5 mass%.

### (Example 2)

A granular catalyst 2 (hereinafter, called a "granulated catalyst 2") including cerium-containing crystalline aluminosilicate was obtained in the same manner as in Example 1, except that 30 g of proton-type crystalline aluminosilicate that had an MFI structure and a molar ratio of silicon/aluminum (Si/Al ratio) of 35 was caused to contain 5 mass% (value calculated when the total mass of the crystalline aluminosilicate is regarded as being 100 mass%) of cerium. In the granulated catalyst 2, the Si/Al ratio was 35, and the amount of cerium (based on the total amount of the catalyst) was 5 mass%.

### (Example 3)

30 g of proton-type crystalline aluminosilicate that had an MFI structure and a molar ratio of silicon/aluminum (Si/Al ratio) of 35 was impregnated with an aqueous lanthanum (III) nitrate solution such that 2.4 mass% (value calculated when the total mass of the crystalline aluminosilicate is regarded as being 100 mass%) of lanthanum was contained in the proton-type crystalline aluminosilicate, and the resultant was heated at 80°C under stirring to cause ion exchange between lanthanum ions and protons of aluminum, followed by drying at 120°C. Thereafter, the resultant was calcined for 3 hours at 780°C under an air flow, thereby obtaining a catalyst including lanthanum-containing crystalline aluminosilicate. Subsequently, tablets were formed in the same manner as in Example 1, thereby obtaining a granular catalyst 3 (hereinafter, called a "granulated catalyst 3"). In the granulated catalyst 3, the Si/Al ratio was 35, and the amount of lanthanum (based on the total amount of the catalyst) was 2.4 mass%.

### (Example 4)

A granular catalyst 4 (hereinafter, called a "granulated catalyst 4") including lanthanum-containing crystalline aluminosilicate was obtained in the same manner as in Example 3, except that 30 g of proton-type crystalline aluminosilicate that had an MFI structure and a molar ratio of silicon/aluminum (Si/Al ratio) of 35 was caused to contain 4.8 mass% (value calculated when the total mass of the crystalline aluminosilicate is regarded as being 100 mass%) of lanthanum. In the granulated catalyst 4, the Si/Al ratio was 35, and the amount of lanthanum (based on the total amount of the catalyst) was 4.8 mass%.

### (Example 5)

30 g of proton-type crystalline aluminosilicate that had an MFI structure and a molar ratio of silicon/aluminum (Si/Al ratio) of 35 was caused to contain 1 mass% (value calculated when the total mass of the crystalline aluminosilicate is regarded as being 100 mass%) of cerium, followed by drying at 120°C. Thereafter, the resultant was calcined for 3 hours at 780°C under an air flow, thereby obtaining cerium-containing crystalline aluminosilicate.
31 g of the obtained cerium-containing crystalline aluminosilicate was impregnated with 30 g of an aqueous diammonium hydrogen phosphate solution such that 0.7 mass% (value calculated when the total weight of the catalyst is regarded as being 100 mass%) of phosphorus was contained in the cerium-containing crystalline aluminosilicate, followed by drying at 120°C. Thereafter, the resultant was calcined for 3 hours at 780°C, thereby obtaining a catalyst containing crystalline aluminosilicate, cerium, and phosphorus.
A pressure of 39.2 MPa (400 kgf) was applied to the obtained catalyst to form tablets, and the resultant was coarsely pulverized to have a size of 20 to 28 mesh (average particle size of 0.65 to 0.85 mm), thereby obtaining a granular catalyst 5 (hereinafter, called a "granulated catalyst 5"). In the granulated catalyst 5, the Si/Al ratio was 35, the amount of cerium (based on the total amount of the catalyst) was 1 mass%, and the amount of phosphorus (based on the total amount of the catalyst) was 0.7 mass%.

### (Example 6)

A granular catalyst 6 (hereinafter, called a "granulated catalyst 6") including cerium-containing crystalline aluminosilicate was obtained in the same manner as in Example 1, except that 30 g of proton-type crystalline aluminosilicate that had an MFI structure and a molar ratio of silicon/aluminum (Si/Al ratio) of 15 was caused to contain 5.8 mass% (value calculated when the total mass of the crystalline aluminosilicate is regarded as being 100 mass%) of cerium. In the granulated catalyst 6, the Si/Al ratio was 15, and the amount of cerium (based on the total amount of the catalyst) was 5.8 mass%.

### (Example 7)

A granular catalyst 7 (hereinafter, called a "granulated catalyst 7") including lanthanum-containing crystalline aluminosilicate was obtained in the same manner as in Example 3, except that 30 g of proton-type crystalline aluminosilicate that had an MFI structure and a molar ratio of silicon/aluminum (Si/Al ratio) of 15 was caused to contain 5.6 mass% (value calculated when the total mass of the crystalline aluminosilicate is regarded as being 100 mass%) of lanthanum. In the granulated catalyst 7, the Si/Al ratio was 15, and the amount of lanthanum (based on the total amount of the catalyst) was 5.6 mass%.

### (Example 8)

A granular catalyst 8 (hereinafter, called a "granulated catalyst 8") including cerium-containing crystalline aluminosilicate was obtained in the same manner as in Example 1, except that 30 g of proton-type crystalline aluminosilicate that had an MFI structure and a molar ratio of silicon/aluminum (Si/Al ratio) of 200 was caused to contain 0.43 mass% (value calculated when the total mass of the crystalline aluminosilicate is regarded as being 100 mass%) of cerium. In the granulated catalyst 8, the Si/Al ratio was 200, and the amount of cerium (based on the total amount of the catalyst) was 0.43 mass%.

### (Example 9)

30 g of proton-type crystalline aluminosilicate that has an MFI structure and a molar ratio of silicon/aluminum (Si/Al ratio) of 35 was caused to contain 2.5 mass% (value calculated when the total mass of the crystalline aluminosilicate is regarded as being 100 mass%) of cerium, followed by drying at 120°C. Thereafter, the resultant was calcined for 3 hours at 780°C, thereby obtaining cerium-containing crystalline aluminosilicate.
32.5 g of the obtained cerium-containing crystalline aluminosilicate was impregnated with 31 g of an aqueous gallium nitrate solution such that 0.2 mass% (value calculated when the total weight of the catalyst is regarded as being 100 mass%) of gallium was contained in the cerium-containing crystalline aluminosilicate, followed by drying at 120°C. Thereafter, the resultant was calcined for 3 hours at 780°C under an air flow, thereby obtaining a catalyst containing crystalline aluminosilicate, cerium, and gallium.
A pressure of 39.2 MPa (400 kgf) was applied to the obtained catalyst to form tablets, and the resultant was coarsely pulverized to have a size of 20 to 28 mesh (average particle size of 0.65 to 0.85 mm), thereby obtaining a granular catalyst 9 (hereinafter, called a "granulated catalyst 9"). In the granulated catalyst 9, the Si/Al ratio was 35, the amount of gallium (based on the total amount of the catalyst) was 0.2 mass%, and the amount of cerium (based on the total amount of the catalyst) was 2.5 mass%.

### (Example 10)

A mixed solution containing 104 g of sodium silicate (J sodium silicate No. 3, SiO₂: 28 to 30 mass%, Na: 9 to 10 mass%, balance: water, manufactured by Nippon Chemical Industrial Co., LTD.) and pure water was added dropwise to diluted sulfuric acid, thereby preparing an aqueous silica sol solution (SiO₂ concentration of 10.2 mass%) as a binder. Meanwhile, distilled water was added to 20.0 g of the catalyst that was obtained in Example 1 and contained crystalline aluminosilicate and cerium, thereby preparing zeolite slurry. The zeolite slurry was mixed with 292 g of the aqueous silica sol solution, and 200 g of an aqueous cerium (III) nitrate solution was added thereto such that 4 mass% (value calculated when the total weight of the catalyst is regarded as being 100 mass%) of cerium was contained in the slurry. The prepared slurry was spray-dried at 250°C, thereby obtaining a spherical catalyst. Thereafter, the catalyst was calcined for 3 hours at 600°C, thereby obtaining a catalyst 1 having a powder form (hereinafter, called a "powdery catalyst 1 ") that contained a silica binder having an average particle size of 85 µm and a bulk density of 0.76 g/cc.
In addition, the amount of cerium (based on the total amount of the catalyst) in the powdery catalyst 1 was 5 mass%.

### (Example 11)

A mixed solution containing 105 g of sodium silicate (J sodium silicate No. 3, SiO₂: 28 to 30 mass%, Na: 9 to 10 mass%, balance: water, manufactured by Nippon Chemical Industrial Co., LTD.) and pure water was added dropwise to diluted sulfuric acid, thereby preparing an aqueous silica sol solution (SiO₂ concentration of 10.2 mass%) as a binder. Meanwhile, distilled water was added to 19.8 g of the catalyst that was obtained in Example 3 and contained crystalline aluminosilicate and lanthanum, thereby preparing zeolite slurry. The zeolite slurry was mixed with 298 g of the aqueous silica sol solution, and 198 g of an aqueous lanthum (III) nitrate solution was added thereto such that 4 mass% (value calculated when the total weight of the catalyst is regarded as being 100 mass%) of lanthanum was contained in the slurry. The prepared slurry was spray-dried at 250°C, thereby obtaining a spherical catalyst. Thereafter, the catalyst was calcined for 3 hours at 600°C, thereby obtaining a catalyst 2 having a powder form (hereinafter, called a "powdery catalyst 2") that contained a silica binder having an average particle size of 84 µm and a bulk density of 0.75 g/cc.
In addition, the amount of lanthanum (based on the total amount of the catalyst) in the powdery catalyst 2 was 5 mass%.

### (Example 12)

A mixed solution containing 105 g of sodium silicate (J sodium silicate No. 3, SiO₂: 28 to 30 mass%, Na: 9 to 10 mass%, balance: water, manufactured by Nippon Chemical Industrial Co., LTD.) and pure water was added dropwise to diluted sulfuric acid, thereby preparing an aqueous silica sol solution (SiO₂ concentration of 10.2 mass%) as a binder. Meanwhile, distilled water was added to 20.2 g of the catalyst that was obtained in Example 1 and contained crystalline aluminosilicate and cerium, thereby preparing zeolite slurry. The zeolite slurry was mixed with 296 g of the aqueous silica sol solution, and 200 g of an aqueous cerium (III) nitrate solution was added thereto such that 24 mass% (value calculated when the total weight of the catalyst is regarded as being 100 mass%) of cerium was contained in the slurry. The prepared slurry was spray-dried at 250°C, thereby obtaining a spherical catalyst. Thereafter, the catalyst was calcined for 3 hours at 600°C, thereby obtaining a catalyst 3 having a powder form (hereinafter, called a "powdery catalyst 3") that contained a silica binder having an average particle size of 85 µm and a bulk density of 0.76 g/cc.

In addition, the amount of cerium (based on the total amount of the catalyst) in the powdery catalyst 3 was 25 mass%.

### (Example 13)

A mixed solution containing 108 g of sodium silicate (J sodium silicate No. 3, SiO₂: 28 to 30 mass%, Na: 9 to 10 mass%, balance: water, manufactured by Nippon Chemical Industrial Co., LTD.) and pure water was added dropwise to diluted sulfuric acid, thereby preparing an aqueous silica sol solution (SiO₂ concentration of 10.2 mass%) as a binder. Meanwhile, distilled water was added to 20.7 g of the catalyst that was obtained in Example 3 and contained crystalline aluminosilicate and lanthanum, thereby preparing zeolite slurry. The zeolite slurry was mixed with 294 g of the aqueous silica sol solution, and 200 g of an aqueous lanthanum (III) nitrate solution was added thereto such that 24 mass% (value calculated when the total weight of the catalyst is regarded as being 100 mass%) of lanthanum was contained in the slurry. The prepared slurry was spray-dried at 250°C, thereby obtaining a spherical catalyst. Thereafter, the catalyst was calcined for 3 hours at 600°C, thereby obtaining a catalyst 4 having a powder form (hereinafter, called a "powdery catalyst 4") that contained a silica binder having an average particle size of 84 µm and a bulk density of 0.75 g/cc.
In addition, the amount of lanthanum (based on the total amount of the catalyst) in the powdery catalyst 4 was 25 mass%.

### (Example 14)

A mixed solution containing 106 g of sodium silicate (J sodium silicate No. 3, SiO₂: 28 to 30 mass%, Na: 9 to 10 mass%, balance: water, manufactured by Nippon Chemical Industrial Co., LTD.) and pure water was added dropwise to diluted sulfuric acid, thereby preparing an aqueous silica sol solution (SiO₂ concentration of 10.2 mass%) as a binder. Meanwhile, distilled water was added to 20.2 g of the catalyst that was obtained in Example 5 and contained crystalline aluminosilicate, cerium, and phosphorus, thereby preparing zeolite slurry. The zeolite slurry was mixed with 300 g of the aqueous silica sol solution, 200 g of an aqueous cerium (III) nitrate solution was added thereto such that 19.6 mass% (value calculated when the total weight of the catalyst is regarded as being 100 mass%) of cerium was contained in the slurry, and 30 g of an aqueous diammonium hydrogen phosphate solution was added thereto such that 0.42 mass% (value calculated when the total weight of the catalyst is regarded as being 100 mass%) of phosphorus was contained in the slurry. The prepared slurry was spray-dried at 250°C, thereby obtaining a spherical catalyst. Thereafter, the catalyst was calcined for 3 hours at 600°C, thereby obtaining a catalyst 5 having a powder form (hereinafter, called a "powdery catalyst 5") that contained a silica binder having an average particle size of 85 µm and a bulk density of 0.75 g/cc.
In addition, in the powdery catalyst 5, the amount of cerium (based on the total amount of the catalyst) was 20 mass%, and the amount of phosphorus (based on the total amount of the catalyst) was 0.7 mass%.

### (Comparative example 1)

A granular catalyst 10 (hereinafter, called a "granulated catalyst 10") including cerium-supported crystalline aluminosilicate was obtained in the same manner as in Example 1, except that 30 g of proton-type crystalline aluminosilicate which had an MFI structure and a molar ratio of silicon/aluminum (Si/Al ratio) of 35 was caused to contain 12 mass% (value calculated when the total mass of the crystalline aluminosilicate is regarded as being 100 mass%) of cerium. In the granulated catalyst 10, the Si/Al ratio was 35, and the amount of cerium (based on the total amount of the catalyst) was 12 mass%. Moreover, the amount of cerium (expressed in terms of the element) based on the crystalline aluminosilicate was 12 mass%.

### (Comparative example 2)

Proton-type crystalline aluminosilicate that had an MFI structure and a molar ratio of silicon/aluminum (Si/Al ratio) of 35 was employed as a granular catalyst 11 (hereinafter, called a "granulated catalyst 11 ").

### (Comparative example 3)

A mixed solution containing 110 g of sodium silicate (J sodium silicate No. 3, SiO₂: 28 to 30 mass%, Na: 9 to 10 mass%, balance: water, manufactured by Nippon Chemical Industrial Co., LTD.) and pure water was added dropwise to diluted sulfuric acid, thereby preparing an aqueous silica sol solution (SiO₂ concentration of 10.2 mass%) as a binder. Meanwhile, distilled water was added to 20.7 g of the catalyst that was obtained in Example 1 and contained crystalline aluminosilicate and cerium, thereby preparing zeolite slurry. The zeolite slurry was mixed with 302 g of the aqueous silica sol solution, and 300 g of an aqueous cerium (III) nitrate solution was added thereto such that 39 mass% (value calculated when the total weight of the catalyst is regarded as being 100 mass%) of cerium was contained in the slurry. The prepared slurry was spray-dried at 250°C, thereby obtaining a spherical catalyst. Thereafter, the catalyst was calcined for 3 hours at 600°C, thereby obtaining a catalyst 6 having a powder form (hereinafter, called a "powdery catalyst 6") that contained a silica binder having an average particle size of 88 µm and a bulk density of 0.77 g/cc.
In addition, in the powdery catalyst 6, the amount of cerium (based on the total amount of the catalyst) was 40 mass%.

### <Evaluation>

### [Measurement of yield of monocyclic aromatic hydrocarbons at the initial stage of reaction: Evaluation 1]

The obtained granulated catalysts 1 to 11 were used to evaluate the catalytic activity at the initial stage of reaction in the following manner.
By using a circulation-type reaction apparatus including a reactor filled with the granulated catalyst (10 ml), the oil feedstock having properties shown in Table 1 was brought into contact with the granulated catalyst and reacted, under the conditions of a reaction temperature: 550°C and a reaction pressure: 0 MPaG. At this time, nitrogen as a diluent was introduced into the apparatus such that oil feedstock came into contact with the granulated catalyst for 7 seconds.
The reaction was allowed to proceed for 30 minutes under the above conditions, thereby producing monocyclic aromatic hydrocarbons having 6 to 8 carbon number. By using an FID gas chromatograph directly connected to the reaction apparatus, the composition of the product was analyzed. The composition of produced liquid was analyzed by using the FID gas chromatograph to evaluate the yield of monocyclic aromatic hydrocarbons at the initial stage of reaction. The measurement results are shown in Table 2.

### [Measurement of yield of monocyclic aromatic hydrocarbons in a pseudo-normal state: Evaluation 2]

Each of the obtained granulated catalysts 1 to 11 was subjected to hydrothermal treatment at a treatment temperature of 650°C for a treatment time of 6 hours in an environment of 100 mass% of vapor, thereby obtaining pseudo-deteriorated granulated catalysts 1 to 11 in a pseudo-normal state that had been caused to undergo pseudo-hydrothermal deterioration. Use of the hydrothermally deteriorated catalyst made it possible to evaluate the yield of monocyclic aromatic hydrocarbons in a pseudo-normal state.
The oil feedstock was reacted in the same manner as in Evaluation 1, except that the above pseudo-deteriorated granulated catalysts were used respectively instead of the granulated catalysts. The composition of the obtained product was analyzed to measure the yield of monocyclic aromatic hydrocarbons. The measurement results are shown in Table 4.

### [Measurement of yield of monocyclic aromatic hydrocarbons at the initial stage of reaction: Evaluation 3]

The obtained powdery catalysts 1 to 6 were used to evaluate the catalytic activity at the initial stage of reaction in the following manner.
By using a circulation-type reaction device including a reactor filled with the powdery catalyst (400 g), the oil feedstock having properties shown in Table 1 was brought into contact with the powdery catalyst and reacted, under the conditions of a reaction temperature: 550°C and a reaction pressure: 0.1 MPaG. At this time, the powdery catalyst was filled in a reaction tube having a diameter of 60 mm, and nitrogen as a diluent was introduced into the device such that oil feedstock came into contact with the powdery catalyst for 10 seconds.
The reaction was allowed to proceed for 10 minutes under the above conditions, thereby producing monocyclic aromatic hydrocarbons having 6 to 8 carbon number. By using an FID gas chromatograph directly connected to the reaction apparatus, the composition of the product was analyzed to measure the yield of monocyclic aromatic hydrocarbons at the initial stage of reaction. The measurement results are shown in Table 3.

### [Measurement of yield of monocyclic aromatic hydrocarbons in a pseudo-normal state: Evaluation 4]

The powdery catalysts 1 to 6 were subjected to hydrothermal treatment at a treatment temperature of 650°C for a treatment time of 6 hours in an environment of 100 mass% of vapor, thereby obtaining pseudo-deteriorated powdery catalysts 1 to 6 in a pseudo-normal state that had been caused to undergo pseudo-hydrothermal deterioration.
The oil feedstock was reacted in the same manner as in Evaluation 3, except that the pseudo-deteriorated powdery catalysts 1 to 6 were used instead of the powdery catalysts 1 to 6. The composition of the obtained product was analyzed to measure the yield of monocyclic aromatic hydrocarbons after hydrothermal deterioration. The measurement results are shown in Table 5.

**[Table 1]**

| Properties of raw material | | | | Method of analysis |
|---|---|---|---|---|
| Density (measured at 15°C) | | g/cm³ | 0.908 | JIS K 2249 |
| Kinematic viscosity (measured at 30°C) | | mm²/s | 3.645 | JIS K 2283 |
| Distillation properties | Initial boiling point | °C | 177.5 | JIS K 2254 |
| | 10 volume% distillation temperature | °C | 226.5 | |
| | 50 volume% distillation temperature | °C | 276.0 | |
| | 90 volume% distillation temperature | °C | 350.0 | |
| | Endpoint | °C | 377.0 | |
| Composition analysis | Saturated fraction | volume% | 34 | JPI-5S-49 |
| | Olefin fraction | volume% | 8 | |
| | Total aromatic fraction | volume% | 58 | |
| | Monocyclic aromatic fraction | volume% | 23 | |
| | Bicyclic aromatic fraction | volume% | 26 | |
| | Aromatic fraction having 3 or more rings | volume% | 9 | |

**[Table 2]**

| | Catalyst | Si/Al ratio of crystalline aluminosilicate | Type of rare earth element | Amount of rare earth element based on crystalline aluminosilicate* (mass%) | Amount of rare earth element based on weight of catalyst* (mass%) | Amount of phosphorus based on weight of catalyst (mass%) | Yield of monocyclic aromatic hydrocarbons (mass%) |
|---|---|---|---|---|---|---|---|
| Example 1 | Granulated catalyst 1 | 35 | Ce | 2.5 | 2.5 | 0 | 37 |
| Example 2 | Granulated catalyst 2 | 35 | Ce | 5 | 5 | 0 | 31 |
| Example 3 | Granulated catalyst 3 | 35 | La | 2.4 | 2.4 | 0 | 36 |
| Example 4 | Granulated catalyst 4 | 35 | La | 4.8 | 4.8 | 0 | 32 |
| Example 5 | Granulated catalyst 5 | 35 | Ce | 1 | 1 | 0.7 | 39 |
| Example 6 | Granulated catalyst 6 | 15 | Ce | 5.8 | 5.8 | 0 | 36 |
| Example 7 | Granulated catalyst 7 | 15 | La | 5.6 | 5.6 | 0 | 37 |
| Example 8 | Granulated catalyst 8 | 200 | Ce | 0.43 | 0.43 | 0 | 29 |
| Example 9 | Granulated catalyst 9 | 35 | Ce | 2.5 | 2.5 | 0 | 39 |
| Comparative example 1 | Granulated catalyst 10 | 35 | Ce | 12 | 12 | 0 | 9 |
| Comparative example 2 | Granulated catalyst 11 | 35 | - | 0 | 0 | 0 | 23 |

| | | | | | | | |
|---|---|---|---|---|---|---|---|
| * expressed in terms of rare earth element | | | | | | | |

**[Table 3]**

| | Catalyst | Si/Al ratio of crystalline aluminosilicate | Type of rare earth element | Amount of rare earth element based on weight of catalyst* (mass%) | Content phosphorus based on weight of catalyst* (mass%) | Yield of monocyclic aromatic hydrocarbons (mass%) |
|---|---|---|---|---|---|---|
| Example 10 | Powdery catalyst 1 | 35 | Ce | 5 | 0 | 36 |
| Example 11 | Powdery catalyst 2 | 35 | La | 5 | 0 | 35 |
| Example 12 | Powdery catalyst 3 | 35 | Ce | 25 | 0 | 33 |
| Example 13 | Powdery catalyst 4 | 35 | La | 25 | 0 | 32 |
| Example 14 | Powdery catalyst 5 | 35 | Ce | 20 | 0.7 | 34 |
| Comparative example 3 | Powdery catalyst 6 | 35 | Ce | 40 | 0 | 25 |

| | | | | | | |
|---|---|---|---|---|---|---|
| * expressed in terms of rare earth element | | | | | | |

**[Table 4]**

| | Catalyst | Si/Al ratio of crystalline aluminosilicate | Type of rare earth element | Amount of rare earth element based on crystalline alummosilicate* (mass%) | Amount of rare earth element based on weight of catalyst* (mass%) | Amount of phosphorus based on weight of catalyst (mass%) | Yield of monocyclic aromatic hydrocarbons (mass%) |
|---|---|---|---|---|---|---|---|
| Example 1 | Pseudo-deteri orated granulated catalyst 1 | 35 | Ce | 2.5 | 2.5 | 0 | 30 |
| Example 2 | Pseudo-deteriorated granulated catalyst 2 | 35 | Ce | 5 | 5 | 0 | 26 |
| Example 3 | Pseudo-deteriorated granulated catalyst 3 | 35 | La | 2.4 | 2.4 | 0 | 30 |
| Example 4 | Pseudo-deteriorated granulated catalyst 4 | 35 | La | 4.8 | 4.8 | 0 | 26 |
| Example 5 | Pseudo-deteriorated granulated catalyst 5 | 35 | Ce | 1 | 1 | 0.7 | 31 |
| Example 6 | Pseudo-deteriorated granulated catalyst 6 | 15 | Ce | 5.8 | 5.8 | 0 | 32 |
| Example 7 | Pseudo-deteriorated granulated catalyst 7 | 15 | La | 5.6 | 5.6 | 0 | 37 |
| Example 8 | Pseudo-deteriorated granulated catalyst 8 | 200 | Ce | 0.43 | 0.43 | 0 | 25 |
| Example 9 | Pseudo-deteriorated granulated catalyst 9 | 35 | Ce | 2.5 | 2.5 | 0 | 31 |
| Comparative example 1 | Pseudo-deteriorated granulated catalyst 10 | 35 | Ce | 12 | 12 | 0 | 5 |
| Comparative example 2 | Pseudo-deteriorated granulated catalyst 11 | 35 | - | 0 | 0 | 0 | 8 |

| | | | | | | | |
|---|---|---|---|---|---|---|---|
| * expressed in terms of rare earth element | | | | | | | |

**[Table 5]**

| | Catalyst | Si/Al ratio of crystalline aluminosilicate | Type of rare earth element | Amount of rare earth element based on weight of catalyst* (mass%) | Content phosphorus based on weight of catalyst (mass%) | Yield of monocyclic aromatic hydrocarbons (mass%) |
|---|---|---|---|---|---|---|
| Example 10 | Pseudo-deteriorated Powdery catalyst 1 | 35 | Ce | 5 | 0 | 24 |
| Example 11 | Pseudo-deteriorated Powdery catalyst 2 | 35 | La | 5 | 0 | 24 |
| Example 12 | Pseudo-deteriorated Powdery catalyst 3 | 35 | Ce | 25 | 0 | 25 |
| Example 13 | Pseudo-deteriorated Powdery catalyst 4 | 35 | La | 25 | 0 | 26 |
| Example 14 | Pseudo-deteriorated Powdery catalyst 5 | 35 | Ce | 20 | 0.7 | 26 |
| Comparative example 3 | Pseudo-deteriorated Powdery catalyst 6 | 35 | Ce | 40 | 0 | 7 |

| | | | | | | |
|---|---|---|---|---|---|---|
| * expressed in terms of rare earth metal | | | | | | |

### <Results>

The yield (mass%) of monocyclic aromatic hydrocarbons of Examples 1 to 9 using the cerium- or lanthanum-containing granulated catalysts 1 to 9 in which the amount of cerium or lanthanum expressed in terms of the element was 0.43 to 5.8 mass% based on crystalline aluminosilicate was 37, 31, 36, 32, 39, 36, 37, 29, and 39, respectively. In addition, the yield (mass%) of monocyclic aromatic hydrocarbons of Comparative example 1 using the granulated catalyst 10 in which the amount of cerium was 12 mass% based on crystalline aluminosilicate in terms of the element and the Comparative example 2 using the granulated catalyst 11 as crystalline aluminosilicate not containing cerium or lanthanum was 9 and 23, respectively. Accordingly, it was found that the yield of monocyclic aromatic hydrocarbons is better in Examples 1 to 9 than in Comparative examples 1 and 2.

The yield of monocyclic aromatic hydrocarbons of Examples 10 to 13 using the powdery catalysts 1 to 4 in which the amount of cerium or lanthanum expressed in terms of the element was 5 to 25 mass% based on the total weight of the catalyst was 36, 35, 33, and 32, respectively, and the yield of monocyclic aromatic hydrocarbons of Example 14 using the powdery catalyst 5 containing cerium and phosphorus was 34 mass%. On the other hand, the yield of monocyclic aromatic hydrocarbons of Comparative example 3 using the powdery catalyst 6 in which the amount of cerium expressed in terms of the element was 40 mass% based on the total weight of the catalyst was 25 mass%. Accordingly, it was found that the yield of monocyclic aromatic hydrocarbons is better in Examples 10 to 14 than in Comparative example 3.

Moreover, the yield (mass%) of monocyclic aromatic hydrocarbons of Examples 1 to 9 using the pseudo-deteriorated granulated catalysts 1 to 9 in which the amount of cerium or lanthanum expressed in terms of the element was 0.43 to 5.8 mass% based on the crystalline aluminosilicate was 30, 26, 30, 26, 31, 32, 37, 25, and 31, respectively. In the examples, the catalytic activity in a pseudo-normal state was excellent, and a desired yield of monocyclic aromatic hydrocarbons was obtained.
On the other hand, the yield of monocyclic aromatic hydrocarbons of Comparative example 1 using the pseudo-deteriorated granulated catalyst 10, which contained a large amount of cerium such that the amount of cerium expressed in terms of the element was 12 mass% based on the crystalline aluminosilicate, was 5 mass%. In this comparative example, the yield of monocyclic aromatic hydrocarbons was low even in a pseudo-normal state. In addition, the yield of monocyclic aromatic hydrocarbons of Comparative example 2 using a pseudo-deteriorated granulated catalyst 11 as crystalline aluminosilicate not containing cerium or lanthanum was 8 mass%. It was found that in this comparative example, the yield of monocyclic aromatic hydrocarbons in a pseudo-normal state markedly decreases, and the catalyst is unpractical since it deteriorates markedly.

In addition, the yield of monocyclic aromatic hydrocarbons of Examples 10 to 13 using the pseudo-deteriorated powdery catalysts 1 to 4 in which the amount of cerium or lanthanum expressed in terms of the element was 5 to 25 mass% based on the weight of the catalyst was 24, 24, 25, and 26 mass%, respectively. Further, the yield of monocyclic aromatic hydrocarbons of Example 14 using the pseudo-deteriorated powdery catalyst 5 containing cerium and phosphorus was 26 mass%. In these examples, the catalytic activity in a pseudo-normal state was excellent, and a desired yield of monocyclic aromatic hydrocarbons was obtained.
On the other hand, the yield of monocyclic aromatic hydrocarbons of Comparative example 3 using the pseudo-deteriorated powdery catalyst 6, which contained a large amount of cerium such that the amount of cerium expressed in terms of the element was 40 mass% based on the weight of the catalyst, was 7 mass%. It was found that in this comparative example, the yield of monocyclic aromatic hydrocarbons decreases markedly, and the catalyst is unpractical since it deteriorates markedly.

So far, preferable embodiments of the present invention have been described, but the present invention is not limited to the above embodiments. Within a scope that is not extrinsic to the object of the present invention, the constitutional elements can be added, omitted, substituted, and modified in another way. The present invention is restricted not by the above description but only by the claims attached.

## Claims

1. A catalyst for producing monocyclic aromatic hydrocarbons that is for producing monocyclic aromatic hydrocarbons having 6 to 8 carbon number from oil feedstock having a 10 volume% distillation temperature of 140°C or higher and a 90 volume% distillation temperature of 380°C or lower, the catalyst comprising:
crystalline aluminosilicate; and
a rare earth element,
wherein the amount of the rare earth element that is expressed in terms of the element is 0.1 to 10 mass% based on the crystalline aluminosilicate.

2. A catalyst for producing monocyclic aromatic hydrocarbons that is for producing monocyclic aromatic hydrocarbons having 6 to 8 carbon number from oil feedstock having a 10 volume% distillation temperature of 140°C or higher and a 90 volume% distillation temperature of 380°C or lower, the catalyst comprising:
crystalline aluminosilicate;
a binder; and
a rare earth element,
wherein the amount of the rare earth element that is expressed in terms of the element is 0.1 to 30 mass% based on the weight of the catalyst.

3. The catalyst for producing monocyclic aromatic hydrocarbons according to Claim 1 or 2, further comprising gallium and/or zinc.

4. The catalyst for producing monocyclic aromatic hydrocarbons according to Claim 3,
wherein in the amount of gallium and/or zinc is 0.05 to 2 mass% based on the crystalline aluminosilicate.

5. The catalyst for producing monocyclic aromatic hydrocarbons according to Claim 3 or 4,
wherein the amount of gallium and/or zinc is 0.02 to 2 mass% based on the weight of the catalyst.

6. The catalyst for producing monocyclic aromatic hydrocarbons according to any one of Claims 1 to 5,
wherein the crystalline aluminosilicate contains phosphorus, and the amount of phosphorus contained in the crystalline aluminosilicate is 0.1 to 3.5 mass% based on the crystalline aluminosilicate.

7. The catalyst for producing monocyclic aromatic hydrocarbons according to any one of Claims 1 to 6, further comprising phosphorus,
wherein the amount of phosphorus is 0.1 to 10 mass% based on the weight of the catalyst.

8. The catalyst for producing monocyclic aromatic hydrocarbons according to any one of Claims 1 to 7,
wherein the rare earth element is at least one kind selected from the group consisting of lanthanum, cerium, praseodymium, neodymium, samarium, gadolinium, and dysprosium.

9. The catalyst for producing monocyclic aromatic hydrocarbons according to any one of Claims 1 to 8,
wherein the crystalline aluminosilicate is medium pore size zeolite.

10. The catalyst for producing monocyclic aromatic hydrocarbons according to any one of Claims 1 to 9,
wherein the crystalline aluminosilicate is MFI type zeolite.

11. A production method of monocyclic aromatic hydrocarbons having 6 to 8 carbon number, comprising:
a step of bringing oil feedstock having a 10 volume% distillation temperature of 140°C or higher and a 90 volume% distillation temperature of 380°C or lower into contact with the catalyst for producing monocyclic aromatic hydrocarbons according to any one of Claims 1 to 10.

12. The production method of monocyclic aromatic hydrocarbons having 6 to 8 carbon number according to Claim 11,
wherein the oil feedstock contains light cycle oil generated by a fluidized catalytic cracking.

13. The production method of monocyclic aromatic hydrocarbons having 6 to 8 carbon number according to Claim 11 or 12,
wherein the oil feedstock is brought into contact with the catalyst for producing monocyclic aromatic hydrocarbons by using a fluidized-bed reaction equipment.
